# EUROPEAN PATENT SPECIFICATION

(11) **EP 4 098 182 B1**
(45) Date of publication and mention of the grant of the patent: **21.01.2026**
(21) Application number: 22177137.1
(22) Date of filing: 03.06.2022
(51) Int. Cl.: A61B 5/024, A61B 5/0205, A61B 5/11, G06F 3/01, G06N 3/08, A61B 5/02, A61B 5/00, G06F 1/16

(54) **MACHINE-LEARNING BASED GESTURE RECOGNITION WITH FRAMEWORK FOR ADDING USER-CUSTOMIZED GESTURES**
AUF MASCHINENLERNEN BASIERENDE GESTENERKENNUNG MIT RAHMEN ZUM HINZUFÜGEN VON BENUTZERSPEZIFISCHEN GESTEN
RECONNAISSANCE DE GESTES BASÉE SUR L'APPRENTISSAGE MACHINE DOTÉE D'UN CADRE POUR L'AJOUT DE GESTES PERSONNALISÉS PAR L'UTILISATEUR

(30) Priority: 04.06.2021 US 202163197307 P; 01.09.2021 US 202163239905 P
(43) Date of publication of application: 07.12.2022
(73) Proprietor: Apple Inc., Cupertino, CA 95014 (US)
(72) Inventor: SEYED MOUSAVI, Hojjat, Cupertino, CA, 95014 (US); SHAHSAVARI, Behrooz, Cupertino, CA, 95014 (US); FERDOSI, Nima, Cupertino, CA, 95014 (US); MAALOUF, Charles, Cupertino, CA, 95014 (US); XU, Xuhai, Cupertino, CA, 95014 (US)
(74) Representative: Lang, Johannes

(56) References cited:
- US-A1- 2019 227 627
- US-A1- 2020 275 895
- US-A1- 2020 356 178
- US-A1- 2021 142 214

## Description

### RELATED APPLICATIONS

This application claims the benefit of priority of U.S provisional patent applications 63/197,307, filed on June 4, 2021, and 63/239,905, filed on September 1, 2021.

### TECHNICAL FIELD

This disclosure relates generally to gesture recognition for navigating user interfaces and performing other actions on wearable devices.

### BACKGROUND

Wearable computers, such as wrist-worn smartwatch, have grown in popularity and are being used for a variety of purposes, such as health monitoring and fitness applications. A user typically interacts with their smartwatch through a touch display and/or crown using hand/finger gestures, such as tap, swipe or pinch. These gestures however, require the user to have a free hand available to perform the gesture. There are many scenarios, however, where a free hand is not available, such as when the user is holding a baby or groceries or if the user is physically disabled.
The prior art document US 2020 / 0 356 178 A1 describes an electronic device which includes a radar transceiver and a processor operably connected to the radar transceiver. The processor is configured to detect a triggering event. In response to detecting the triggering event, the processor is configured to transmit, via the radar transceiver, radar signals. The processor is also configured to identify a gesture from reflections of the radar signals received by the radar transceiver. The processor is further configured to determine whether the gesture is associated with the triggering event. Based on determining that the gesture is associated with the triggering event, the processor is configured to perform an action indicated by the gesture.
The prior art document US 2020 / 0 275 895 A1 describes an apparatus which comprises a processor that is programmed to: receive, via a plurality of neuromuscular sensors, a first plurality of neuromuscular signals from a user as the user performs a first single act of a gesture; train a classification model based on the first plurality of neuromuscular signals, the training including: deriving value(s) from the first plurality of neuromuscular signals, the value(s) indicative of distinctive features of the gesture including at least one feature that linearly varies with a force applied during performance of the gesture; and generating a first categorical representation of the gesture in the classification model based on the value(s); and determine that the user performed a second single act of the gesture, based on the trained classification model and a second plurality of neuromuscular signals.
The prior art document US 2021 / 0 142 214 A1 describes a device which includes at least one processor configured to receive, from a first sensor of the device, first sensor output of a first type, and receive, from a second sensor of the device, second sensor output of a second type that differs from the first type. The at least one processor is further configured to provide the first sensor output and the second sensor output as inputs to a machine learning model, which has been trained to output a predicted gesture based on sensor output of the first type and sensor output of the second type. The at least one processor is further configured to determine the predicted gesture based on an output from the machine learning model, and to perform, in response to determining the predicted gesture, a predetermined action on the device.
The prior art document US 2019 / 0 227 627 A1 describes a method which controls presentation of instructions via a user interface to instruct the user to perform the at least one gesture and updating at least one parameter of the one or more statistical models based, at least in part on a plurality of neuromuscular signals recorded by a plurality of neuromuscular sensors during performance of the at least one gesture by the user.

### SUMMARY

The invention is set out in the appended set of claims. Embodiments are disclosed for machine learning (ML) based gesture recommendation with a framework for adding user-customized gestures.

In an embodiment, a method comprises: receiving sensor data indicative of a gesture made by a user, the sensor data obtained from at least one sensor of a wearable device worn on a limb of the user; generating a current encoding of features extracted from the sensor data using a machine learning model with the features as input; generating similarity metrics between the current encoding and each encoding in a set of previously generated encodings for gestures; generating similarity scores based on the similarity metrics; predicting the gesture made by the user based on the similarity scores; and performing an action on the wearable device or other device based on the predicted gesture.

In an embodiment, the limb is a wrist of the user and the sensor data is obtained from a combination of a biosignal and at least one motion signal.

In an embodiment, the biosignal is a photoplethysmography (PPG) signal and the at least one motion signal is acceleration.

In an embodiment, the similarity metrics are distance metrics.

In an embodiment, the machine learning model is a neural network.

In an embodiment, the similarity scores are predicted by a neural network.

In an embodiment, the neural network used to predict the similarity scores is a deep neural network that includes a sigmoid activation function.

In an embodiment, the action corresponds to navigating a user interface on the wearable device or other device.

In an embodiment, the machine learning model is a neural network trained using sample data for pairs of gestures obtained from a known set of gestures, where each gesture in the pair is annotated with a label indicating that the gesture is from a same class or a different class, and a feature vector for each gesture in the pair is separately extracted and then encoded using the first machine learning model.

In an embodiment, the machine learning model uses a different loss function for each gesture in each pair during training.

Other embodiments can include an apparatus, computing device and non-transitory, computer-readable storage medium.

Particular embodiments described herein provide one or more of the following advantages. A user is free to create a custom input gesture for interacting with a user interface (UI) of a wearable device (e.g., a smartwatch) using one or more data samples collected by sensors of the wearable device without updating the software on the device. To add a customized gesture, the user performs the customized gesture and sensor data resulting from the gesture are captured by motion sensors (e.g., accelerometers, angular rate sensors) and a biosignal sensor of the wearable device, such as a photoplethysmography (PPG). The customized gesture can be added by the user, thus avoiding large-scale data collection to add customized gestures prior to shipping the wearable device.

The captured sensor data is input into an encoder network configured to generate a first encoding of the features (e.g., a feature vector) for the customized gesture. A set (e.g., one or more) of previously generated encodings for at least one other gesture is obtained from memory of the wearable device. A similarity metric (e.g., a distance metric) is computed for all pairwise combinations of the first encoding and the set of encodings, and pairwise similarity score(s) are computed using a ML model trained to predict similarity score(s) between pairs of input gestures. The pair of gestures that is most similar among all the pairs of gestures based on its similarity score is selected as the gesture intended by the user. At least one action (e.g., a predefined action) associated with the selected gesture is initiated or performed on the wearable device or another device in accordance with the selected gesture.

The details of one or more implementations of the subject matter are set forth in the accompanying drawings and the description below. Other features, aspects and advantages of the subject matter will become apparent from the description, the drawings and the claims.

### BRIEF DESCRIPTION OF THE DRAWINGS

FIG. 1 illustrates various types of gestures for navigating a UI or performing other actions on a wearable device, according to an embodiment.
FIG. 2A is a block diagram of a ML-based network for predicting gestures, according to an embodiment.
FIG. 2B is a more detailed block diagram of the ML-based network for predicting gestures, according to an embodiment.
FIG. 3 is block diagram illustrating a training framework for adding a customized gestures, according to an embodiment.
FIG. 4 is a block diagram illustrating an inference framework for adding customized gestures, according to an embodiment.
FIG. 5 is a flow diagram of a process of predicting a customized gesture, according to an embodiment.
FIG. 6 is block diagram of a system architecture for implementing the features and processes described in reference to FIGS. 1-5.
FIG. 7A to 7C is a block diagram of a data processing pipeline for training a gesture customization prediction head, according to an embodiment.

### DETAILED DESCRIPTION

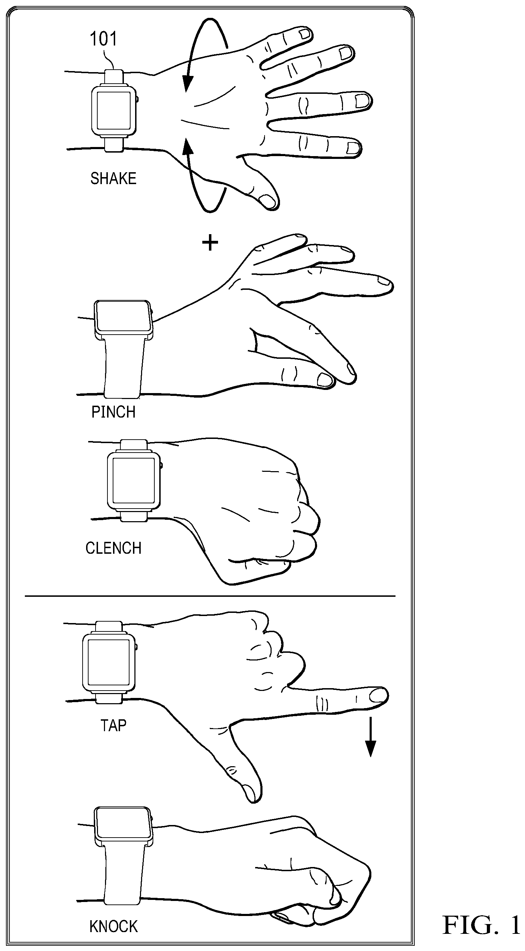

FIG. 1 illustrates various types of example gestures navigating a UI or performing other actions on wearable device 101, according to an embodiment. Wearable device 101 is shown as a smartwatch. Other wearable devices are also applicable to the disclosed embodiments, including devices worn on other user limbs or on the user's face, head or any other body part where muscle contractions and/or movement of the body part can be captured by biosignal sensors and/or motion sensors.

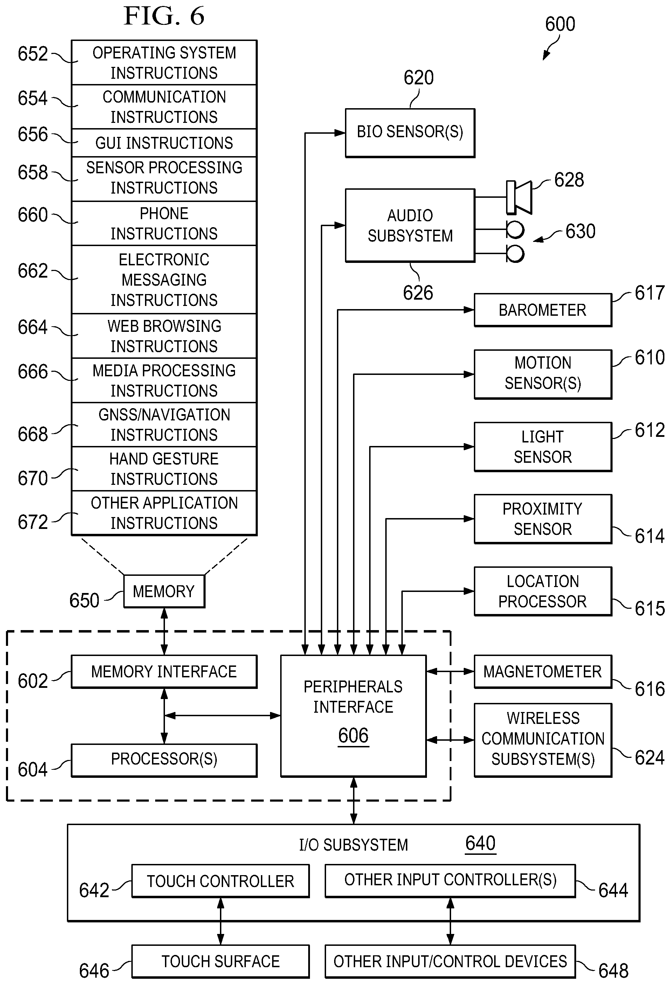

The examples shown in FIG. 1 include but are not limited to: pinching the thumb and index finger together, clenching the hand into a fist, tapping one or more fingers on a surface and knocking a fist on a surface. These gestures generate motion and/or forces that can be captured by the biosignal sensors and/or motion sensors of wearable device 101 (e.g., a smartwatch) shown in FIG. 1. An example wearable device system architecture is described in reference to FIG. 6. Although wearable device 101 is shown as a smartwatch attached to a user's wrist, in other applications the wearable device can be attached to other limbs or parts of limbs (e.g., to a user's arm or leg), or multiple wearable devices can be attached to multiple limbs.

In some embodiments, the biosignal sensor(s) is a PPG sensor configured to detect blood volume changes in microvascular bed of tissue of a user (e.g., where the user is wearing the device on his/her body, such as his/her wrist). The PPG sensor may include one or more light-emitting diodes (LEDs) which emit light and a photodiode/photodetector (PD) which detects reflected light (e.g., light reflected from the wrist tissue). The biosignal sensor(s) are not limited to a PPG sensor, and may additionally or alternatively correspond to one or more of: an electroencephalogram (EEG) sensor, an electrocardiogram (ECG) sensor, an electromyogram (EMG) sensor, a mechanomyogram (MMG) sensor (e.g., piezo resistive sensor) for measuring muscle activity/contractions, an electrooculography (EOG) sensor, a galvanic skin response (GSR) sensor, a magnetoencephalogram (MEG) sensor and/or other suitable sensor(s) configured to measure bio signals.

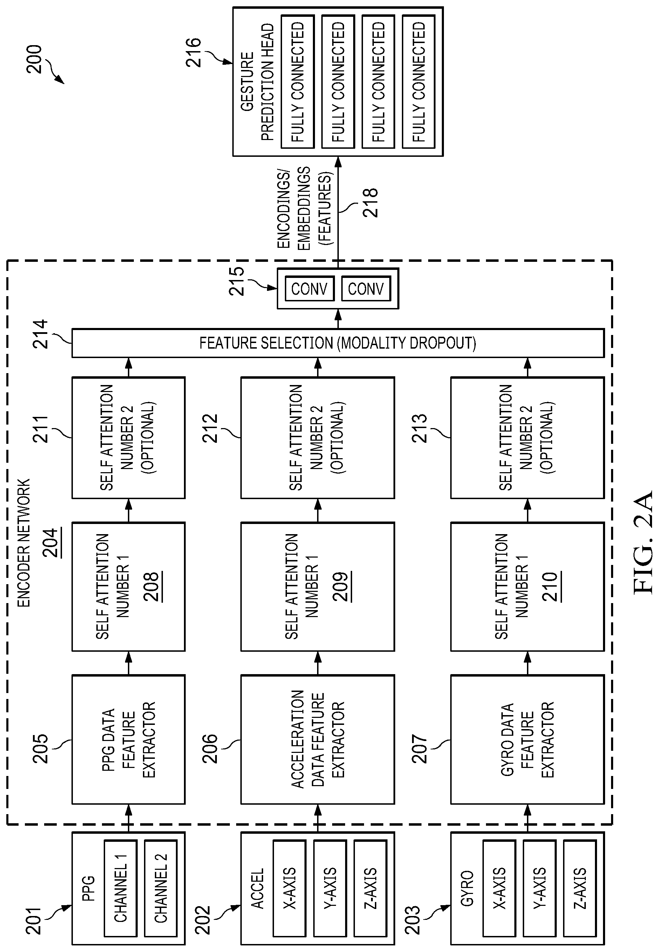

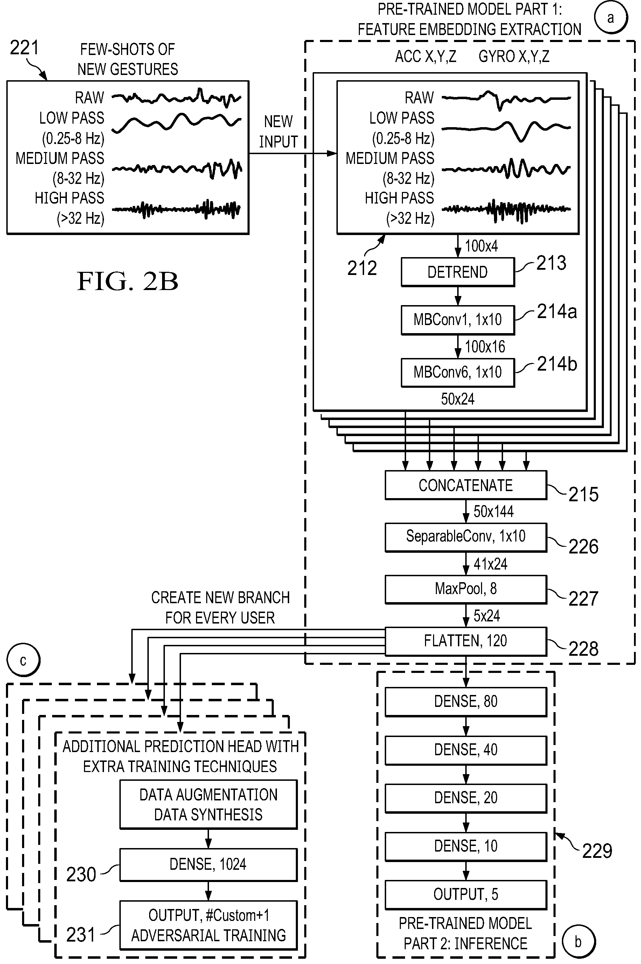

In some embodiments, wearable device 101 includes non-bio signal sensor(s) that include one or more motion sensors for detecting device motion. For example, the motion include but are not limited to accelerometers and angular rate sensors (e.g., gyroscopes) for detecting device acceleration and angular rates, respectively. As discussed further below with respect to FIGS. 2-6, wearable device 101 may be configured to predict a finger/hand gesture based on sensor data provided by the biosignal sensor(s) and/or motion sensor(s).

In the disclosure that follows, ML-based training and inference frameworks are disclosed that allow a user to add one or more customized gestures to a set of previously learned gestures, such as, for example, adding a shaking gesture where the user rotates their hand clockwise and counterclockwise, as shown in FIG. 1.

FIG. 2 is a block diagram of a ML model 200 for predicting gestures, according to an embodiment. ML model 200 is configured to receive sensor data from PPG 201, accelerometers 202 and gyros 203. Other embodiments may include more or fewer sensor data inputs. In one or more implementations, one or more of the sensor data inputs may correspond to a window of time (e.g., 0.5 seconds, 0.1 seconds, or any window of time) in which sensor data was collected by the respective sensor. Moreover, the sensor inputs may be filtered and/or pre-processed (e.g., normalized) before being provided as inputs to ML model 200.

The sensor data is input into encoder network 204, which includes a separate processing path for each sensor modality. In an embodiment, a PPG data path includes PPG data feature extractor 205, self-attention network 208 and self-attention network 211. An accelerometer data path includes acceleration data feature extractor 206, self-attention network 209 and self-attention network 212. A gyro data path includes gyro data feature extractor 207, self-attention network 210 and self-attention network 213. The outputs of each of these data processing paths are combined in feature selector 214, which selects particular features from particular sensor modality processing paths as input into convolution layers 215.

Feature extractors 205, 206, 207 can be implemented using a suitable feature extraction technique, including but not limited to using a convolutional neural network (CNN) for feature extraction. Self-attention networks 208, 213 include a series of convolutional layers and normalization layers (e.g. batch normalization) that are trained to learn which sensor data is most important based on context (e.g., the self-attention networks 208, 213 are to enhance or diminish the input feature prior to prediction. In an embodiment, the self-attention networks 208, 213 are repeated twice to increase the depth of encoder network 204 and the ability of the encoder network 204 to extract more relevant features for gesture prediction head 216. The output of encoder network 204 are feature encodings (e.g., a feature vector) are input into gesture prediction head 216, which includes a fully connected layer for predicting gestures

In some embodiments, gesture prediction head 216 includes a fully connected layer (e.g., a CNN layer or dense layer) to predict a gesture performed by the user. The gesture may correspond to a single-handed gesture performed by the same hand that is coupled to wearable device 101, as shown in FIG. 1. The gesture may correspond to a static gesture (e.g., a specific type of hand/finger positioning that is held for a predefined time period) and/or a dynamic gesture (e.g., a motion-based gesture performed over a predefined time period). Moreover, the gesture may correspond to a finger-based gesture (e.g., in which the fingers move and/or are positioned in a specific manner), a wrist-based gesture (e.g., in which the wrist moves and/or is positioned in a specific manner) and/or a combination of a finger-based and wrist-based gesture. In some embodiments, the gesture may correspond to a gesture performed on a horizontal and/or vertical surface, such as, for example, a table, a wall, a floor, and/or another hand.

In some embodiments, ML model 200 is trained on different device(s) (e.g., one or more smartwatches other than the electronic device based on sensor output data prior to being deployed on wearable device 101. The sensor output data for training may correspond to output from one or more biosignal sensor(s) and/or from one or more non-biosignal sensors (e.g., motion sensors). In some embodiments, ML model 200 is trained across multiple users, for example, who provided different types of gestures while wearing a device (e.g., another smartwatch with biosignal and/or non-biosignal sensor(s)) and confirmed the gestures (e.g., via a training user interface) as part of a training process. In some embodiments, video of the user making the gestures is used to manually or automatically annotate training data for prediction the gestures. In this manner, ML model 200 is trained to predict gestures across a general population of users, rather than one specific user. In some embodiments, the training data is augmented or domain adapted to improve diversification of the training data so that prediction can be made under a variety of environment conditions.

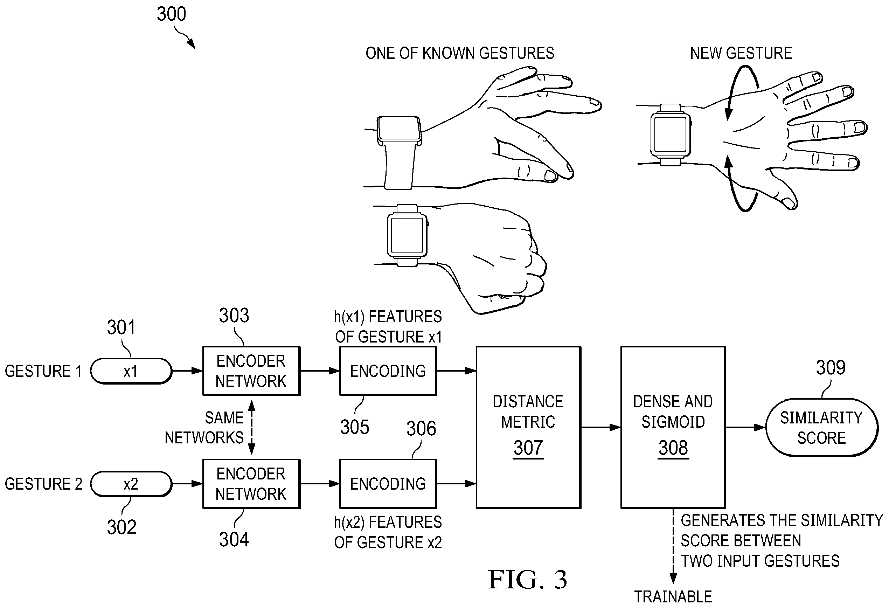

As previously described, it is desirable to allow a user to add customized gestures to an existing set of learned gestures. For example, wearable device 101 may be deployed to users with encoder network 204 trained on the pinch, clench, tap and knock gestures, but not trained on the shake gesture. One solution would be to train encoder network 204 on training data for the shake gesture, as described above, and then deploy an updated encoder network 204 to the installed customer base. This solution, however, can take many months and a decision on which new gesture to add would likely be based on the gesture with the most demand, which may not be desirable to all users. Accordingly, an alternative solution is disclosed that allows the user to add a customized gesture, which described in reference to FIGS. 3-6 below.

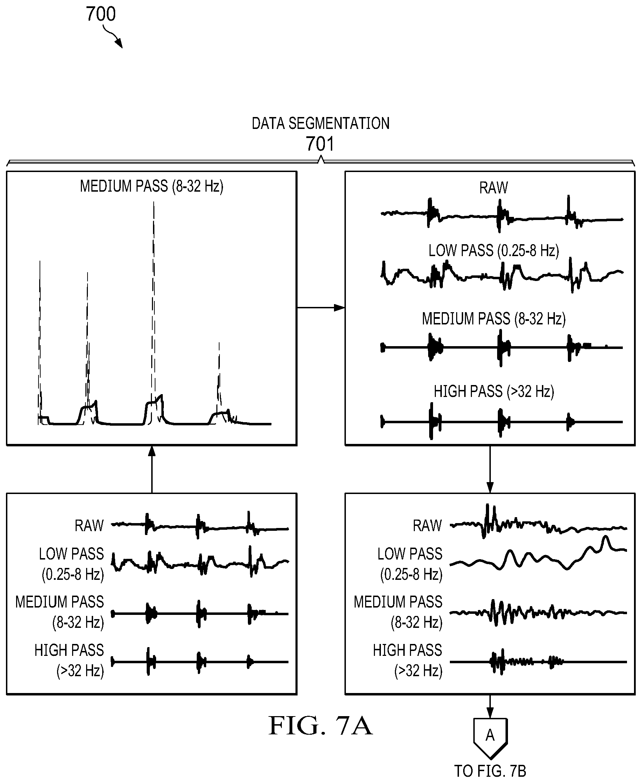

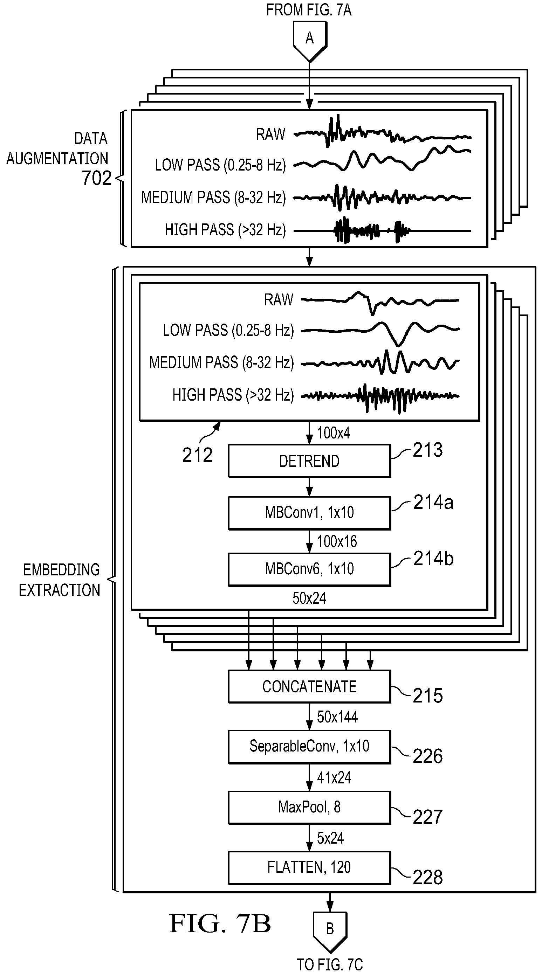

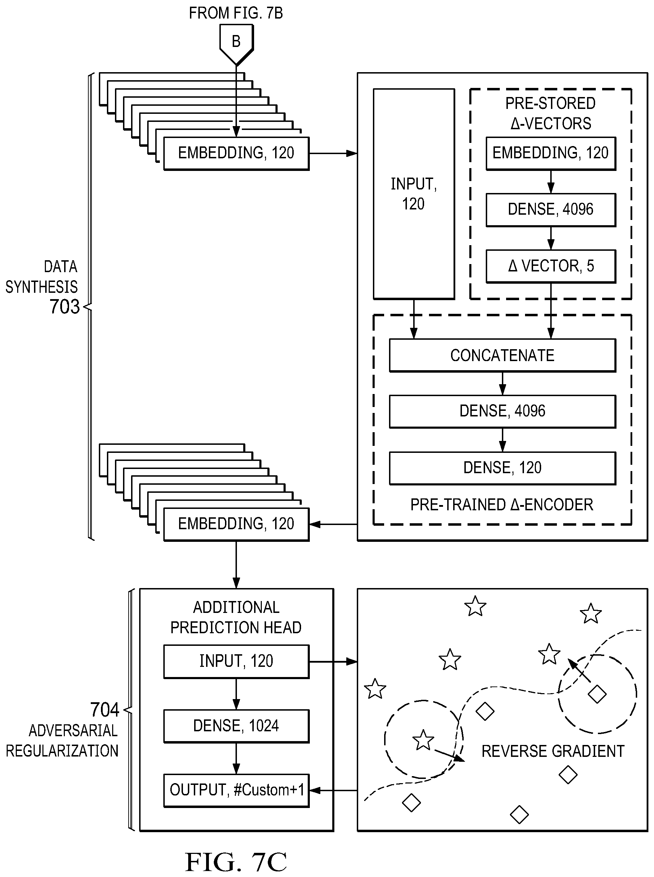

FIG. 2B is a block diagram of an alternative ML-based network 220 for predicting gestures, according to an embodiment. Network 220 integrates transfer learning, class incremental learning and few-shot learning. The upper region shows the two parts of a pre-trained gesture recognition model: feature embedding extraction A and inference B. The lower region shows an additional gesture prediction head C that can be trained on new inputs of new gestures using additional training techniques, as described in reference to FIG. 7. Both the pre-trained model and the additional prediction head C model share the same feature embedding layers.

The raw input of network 220 is n-sec (e.g., 1 sec) 6 degree-of-freedom (DOF) IMU signals 221 (three axes for accelerometer, and three axes for gyroscope) sampled at 100 Hz. In an embodiment, the input signal 221 are preprocessed by respective Butterworth filters 222 (e.g., 0.22-8 Hz, 8-32 Hz, 32 Hz) using cascaded second-order sections, leading to 100 × 4 input 223 for one channel.

The concept of EfficientNet is adopted to balance the number of trainable parameters and model performance, as described in Mingxing Tan and Quoc Le. 2019. EfficientNet: Rethinking Model Scaling for Convolutional Neural Networks. In *International Conference on Machine Learning.* PMLR, 6105-6114. Specifically, for each input channel, two inverted residual blocks are employed (e.g., MBConv 224a, 224b in FIG. 2B) to process the signals. The output of the six channels are concatenated 225, and one more separable convolution layers 226 are used to capture concatenated information with low computational cost, followed by a max-pooling layer 227 and a flatten layer 228. These layers are marked as the feature embedding extraction part of the pre-trained model Part 1 (FIG. 2B), whose output is a one-dimension vector with a length of 120.

The latter half of the pre-trained model consists of a stack of five fully connected layers 229 with sizes as 80, 40, 20, 10, and 5. In an embodiment, a batch normalization layer and a dropout layer (p = 0.5) are inserted between every two fully connected layers to improve model generalizability. The output of the final layers corresponds to the confidence of the five classes. The whole model has 106k parameters. In an embodiment, cross-entropy is used as the loss function, and an Adam optimizer is used during the training.

In an embodiment, the additional gesture prediction head C employs a two-layer fully connected network. The first layer 230 is a feature processing layer and the second layer 231 is an output layer. In an embodiment, the first layer 230 includes leaky ReLU (*α* = 0.3) as the activation function and has a L2 kernel regularizer (*λ* = 5e-5) and a dropout layer (p = 0.5) to reduce overfitting. The second layer 231 uses Softmax activation that corresponds to the prediction confidence of the final classes. The number of the classes is equal to the number of customized gestures plus one more class for the negative case.

Therefore, when users create their first customized gesture, the additional prediction head C is trained as a binary classifier. When a second gesture is added, a new three-class prediction head is trained from scratch, etc. Since the additional prediction head C is light-weighted, the training process is fast. In real-time, the additional prediction head C works together with network 220 to recognize distinctive gestures and are both robust to negative data. In an embodiment, if both predict a gesture, the one with the highest confidence is the final prediction. The framework leverages the first half of the pre-trained model as a feature extractor and transfers the extracted features to new gesture recognition tasks. By training the additional prediction head C for incremental classes, the performance of the existing (default) gestures is not impacted, addressing the forgetting old problem. Then, the few-shot challenge with a series of data processing techniques is performed.

In an embodiment, if a new custom gesture is similar to existing gestures, or performed inconsistently by the user, or close to daily activities of the user, feedback (e.g., through a text display or audio on a smartwatch) can be provided to users with a request to define another gesture. Moreover, if a new/custom gesture is novel and performed consistently, but the model is trained with fair performance, users can be offered to choose finishing or collecting a few more samples. Such a feedback can help users to better understand the process and design gestures.

In an embodiment, a gesture customization process flow is described as follows. A user creates a custom gesture and the smartwatch or other wearable device captures a plurality of shots (e.g., 3 shots) of repetition of the custom gesture performed by the user. The few shots are segmented and pre-analyzed to determine if the gesture is: (1) similar to existing gestures, (2) inconsistent among shots, (3) easily confused gestures performed with daily activities, or (4) a consistent novel gesture. If (1) through (3), the user is asked to define a new gesture. If (4), then the training process will proceed a confidence score for the training is computed. If the confidence score indicates a poor result, then the user is asked to define a new gesture. If the confidence score indicates good confidence, the recording is finished and the new gesture is added to the existing gestures. If the confidence score indicates fair confidence, the user is informed of the fair confidence and they are asked if they want to perform more shots. If the user does not perform more shots, then the recording of gestures terminates and the new gestures are added to the existing gestures. If the user performs more shots, the training continues, the confidence score for the training is computed, and either more shots are recorded or the recording terminates, and so forth.

In an embodiment, the segmented data is feed into the pre-trained model (FIG. 2B) and the additional gesture prediction head C (if it exists). If either model predicts the majority of the segments to be one of the existing gestures, it indicates that the new gesture is close to previous gestures.

During the segmentation, a distance matrix of potential gesture repetitions is checked and those gestures that are far from the rest of the repetitions are filtered out. After the filtering, if the number of repetitions left is less than the expected number (e.g., 3 when the framework requires a 3-shot recording), then the user did not perform the gesture consistently.

To find whether the new gesture is close to common daily behaviors, the negative data collected is leveraged. The pre-trained model (FIG. 2B) is used to extract the embeddings of the negative data in the testing set (sliced in 1-sec pieces), and Hierarchical Density-Based Spatial Clustering of Applications with Noise (HDBSCAN) or other suitable clustering algorithm is applied to automatically cluster the data. HDBSCAN is a variant of DB- SCAN that can adapt different distance thresholds based on the cluster density, obviating the necessity of setting this hyperparameter. In an embodiment, a Euclidean distance is used as the metric, and the minimal cluster size can be set as 3. HDBSCAN identifies multiple clusters. the center of these clusters is determined and saved to be used as a representation of common daily activities. After the new gesture data is segmented, a distance matrix is computed between the gesture data and these cluster centers, and each gesture sample's closest center is determined. If the majority of the gesture data are close to at least one of these centers (e.g., a threshold empirically set as 0.4), then the new gesture is close to common daily activities.

FIG. 3 is block diagram illustrating training framework 300 for adding customized gestures, according to an embodiment. Generally, training framework 300 creates pairs of gestures (X1, X2) from a set of known gestures (e.g., pinch, clench, tap, knock), which in some embodiments can be samples stored in memory of wearable device 101. Similarity labels are generated for each pair. For example, label=1 if X1, X2 are from the same class, or label=0 if X1, X2 are from different classes. In other embodiments, different loss functions can be used for the training of the network, such as, for example, a triple-loss function or quadruple-loss function. As shown in FIG. 3, framework 300 receives as input sensor data for gesture pair X1, X2. The sensor data is input into separate encoder networks 303, 304 trained to generate a separate encoding (e.g., feature vector) for each gesture in the pair. In some embodiments, encoder networks 303, 304 are configured like encoder network 204 shown in FIG. 2. In some embodiments, encoder networks 303, 304 are a Siamese network. Encoder networks 303, 304 output encodings 305, 306, which in this example are feature vectors h(X1), h(X2) embedding various features of the gestures X1, X2. Encodings 305, 306 are input into similarity metric generator 307, which generates a similarity metric for encodings 305, 306, i.e., a similarity metric for the gesture pair. In some embodiments, the similarity metric is a distance metric which measures the distance between the feature vectors h(X1), h(X2). In some embodiments, the distance metric is an element-wise subtraction. In some embodiments, the distance metric is a Euclidean distance metric. Other distance metrics can also be used, such as Manhattan, Minkowski or Chebychev distance metrics. For example, a suitable user interface can be presented on a display of the device to instruct the user on how to register a custom gesture. In some embodiments, the user can register a custom gesture during a registration step on the device implemented by the operating system and/or a utility application. During registration, sensor data is collected while the user makes the gesture and the encoder network is updated to recognize the new custom gesture based on the collected sensor data.

The similarity metric is then input into ML model 308 which is trained to predict a similarity score 309 between two input gestures based on the similarity metric for the pair. In an embodiment, ML model 308 is a deep neural network with one or more dense layers coupled to an activation function that outputs a similarity score (e.g., a probability). In some embodiments, the activation function can be any suitable linear or non-linear activation function. In some embodiments, the activation function is a logistic activation function, such as a sigmoid function or softmax function. In some embodiments, ML model 308 takes the distance metric from metric generator 307 (e.g., a vector of feature differences) and feeds the distance metric into a fully connected layer or layers. The output of ML model 308 is one value that shows the similarity between the two input gestures. If the value is 1, then the input gestures are similar; if the value is zero, then the input gestures are dissimilar.

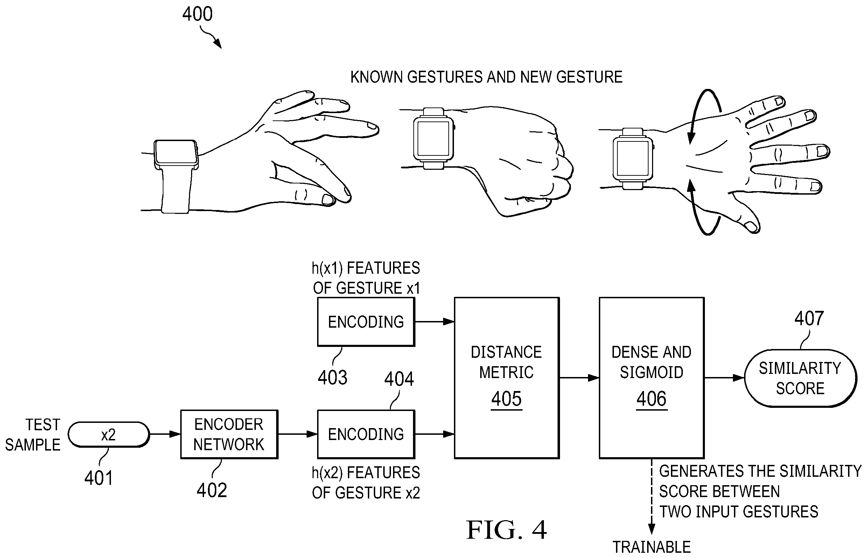

FIG. 4 is a block diagram illustrating use of an inference framework 400 for adding custom gestures, according to an embodiment. After training, encodings 303, 305 can be stored in memory of wearable device 101. For any new sample of sensor data captured when the user performs a current gesture (e.g., a shake gesture), a current encoding 404 (e.g., current feature vector) is generated by encoder network 402 (e.g., same encoder network as encoder networks 204, 303, 304). Similarity metric 405 (e.g., a distance metric) is computed between the current encoding and each of previously generated encodings 403 for gestures stored in, for example, memory of wearable device 101. The similarity metric 405 computed for each gesture pair is input into ML model 406 which predicts similarity score 407, using, for example, a deep neural network coupled to a sigmoid or softmax activation function. The previously stored encoding/gesture with the highest similarity with the current encoding based on the similarity scores for the pairs (e.g., highest probability) determines the predicted user gesture. In this example, the shake gesture would be the predicted user gesture.

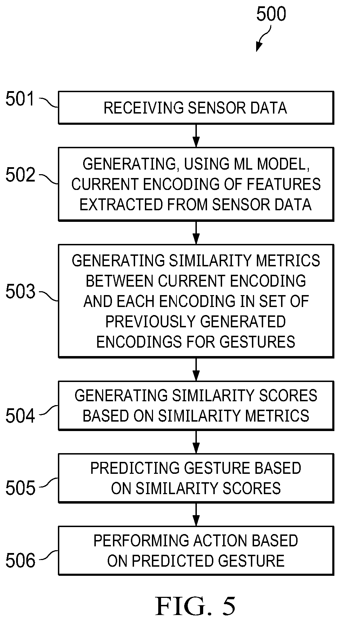

FIG. 5 is a flow diagram of process 500 of inferring a gesture based on sensor data and the encoder network 402 shown in FIG. 4, according to an embodiment. Process 500 can be implemented using the system architecture described in reference to FIG. 6.

Process 500 begin by receiving sensor data indicative of a gesture made by a user (501). The sensor is obtained from at least one sensor of a wearable device (e.g., accelerometer, gyro, PPG) worn on a limb (e.g., wrist) of the user that was used to make the gesture. For example, the gesture can be a customized gesture made by the user while wearing the wearable device on her wrist. The gesture can be, for example, the shake gesture shown in FIG. 1.

Process 500 continues by generating a current encoding of features extracted from the sensor data using ML model with the features as input (502). For example, a neural network can be used as the ML model. In an embodiment, the neural network includes one or more self-attention networks, a described in reference to FIG. 2.

Process 500 continues by generating similarity metrics between the current encoding and each encoding in a set of previously generated encodings for gestures (503). For example, a distance metric can be the similarity metric for measuring a distance between feature vectors for different gesture pairs in a n-dimensional space (e.g., Euclidean distance). Similarity metrics can be computed for pairs of feature vectors: (shake, pinch), (shake, clench), (shake, tap) and (shake, knock), (shake, shake).

Process 500 continues by generating similarity scores based on the similarity metrics (504). For example, a deep neural network can be used to predict the similarity scores based on the similarity metrics. In an embodiment, the deep neural network can be coupled to a sigmoid activation function that outputs probabilities of match.

Process 500 continues by determining a gesture made by the user based on the similarity scores (505). For example, the gesture pair with the highest similarity score (e.g., the highest probability of match) can be selected as the user's gesture. Using the above gesture examples, the pair (shake, shake) would have the highest similarity score. In an embodiment, if none of the pairs are sufficiently close (e.g., all the similarity scores fall below a minimum threshold probability), then no action takes place as the system assumes that the detection bio signals and/or motion signal were not gestures.

Process 500 continues by performing an action on the wearable device or another device based on the determined gesture (506). For example, the determined gesture can be used to navigate a graphical user interface (GUI) presented on a display of the wearable device or otherwise interact with the user interface, perform a desired function such as invoking or closing an application, or initiating and ending a communication modality.

FIG. 6 is block diagram of a system for implementing the features and processes described in reference to FIGS. 1-5 and 7. Architecture 600 can include memory interface 602, one or more hardware data processors, image processors and/or processors 604 and peripherals interface 606. Memory interface 602, one or more processors 604 and/or peripherals interface 606 can be separate components or can be integrated in one or more integrated circuits. System architecture 600 can be included in any suitable electronic device, including but not limited to: a smartwatch, smartphone, fitness band and any other device that can be attached, worn or held by a user.

Sensors, devices and subsystems can be coupled to peripherals interface 606 to provide multiple functionalities. For example, one or more motion sensors 610, light sensor 612 and proximity sensor 614 can be coupled to peripherals interface 606 to facilitate motion sensing (e.g., acceleration, rotation rates), lighting and proximity functions of the wearable device. Location processor 615 can be connected to peripherals interface 606 to provide geo-positioning. In some implementations, location processor 615 can be a GNSS receiver, such as the Global Positioning System (GPS) receiver. Electronic magnetometer 616 (e.g., an integrated circuit chip) can also be connected to peripherals interface 606 to provide data that can be used to determine the direction of magnetic North. Electronic magnetometer 616 can provide data to an electronic compass application. Motion sensor(s) 610 can include one or more accelerometers and/or gyros configured to determine change of speed and direction of movement. Barometer 617 can be configured to measure atmospheric pressure. Biosignal sensor 620 can be one or more of a PPG sensor, an electroencephalogram (EEG) sensor, an electrocardiogram (ECG) sensor, an electromyogram (EMG) sensor, a mechanomyogram (MMG) sensor (e.g., piezo resistive sensor) for measuring muscle activity/contractions, an electrooculography (EOG) sensor, a galvanic skin response (GSR) sensor, a magnetoencephalogram (MEG) sensor and/or other suitable sensor(s) configured to measure biosignals.

Communication functions can be facilitated through wireless communication subsystems 624, which can include radio frequency (RF) receivers and transmitters (or transceivers) and/or optical (e.g., infrared) receivers and transmitters. The specific design and implementation of the communication subsystem 624 can depend on the communication network(s) over which a mobile device is intended to operate. For example, architecture 600 can include communication subsystems 624 designed to operate over a GSM network, a GPRS network, an EDGE network, a Wi-Fi^{™} network and a Bluetooth^{™} network. In particular, the wireless communication subsystems 624 can include hosting protocols, such that the mobile device can be configured as a base station for other wireless devices.

Audio subsystem 626 can be coupled to a speaker 628 and a microphone 630 to facilitate voice-enabled functions, such as voice recognition, voice replication, digital recording and telephony functions. Audio subsystem 626 can be configured to receive voice commands from the user.

I/O subsystem 640 can include touch surface controller 642 and/or other input controller(s) 644. Touch surface controller 642 can be coupled to a touch surface 646. Touch surface 646 and touch surface controller 642 can, for example, detect contact and movement or break thereof using any of a plurality of touch sensitivity technologies, including but not limited to capacitive, resistive, infrared and surface acoustic wave technologies, as well as other proximity sensor arrays or other elements for determining one or more points of contact with touch surface 646. Touch surface 646 can include, for example, a touch screen or the digital crown of a smart watch. I/O subsystem 640 can include a haptic engine or device for providing haptic feedback (e.g., vibration) in response to commands from processor 604. In an embodiment, touch surface 646 can be a pressure-sensitive surface.

Other input controller(s) 644 can be coupled to other input/control devices 648, such as one or more buttons, rocker switches, thumb-wheel, infrared port and USB port. The one or more buttons (not shown) can include an up/down button for volume control of speaker 628 and/or microphone 630. Touch surface 646 or other controllers 644 (e.g., a button) can include, or be coupled to, fingerprint identification circuitry for use with a fingerprint authentication application to authenticate a user based on their fingerprint(s).

In one implementation, a pressing of the button for a first duration may disengage a lock of the touch surface 646; and a pressing of the button for a second duration that is longer than the first duration may turn power to the mobile device on or off. The user may be able to customize a functionality of one or more of the buttons. The touch surface 646 can, for example, also be used to implement virtual or soft buttons.

In some implementations, the mobile device can present recorded audio and/or video files, such as MP3, AAC and MPEG files. In some implementations, the mobile device can include the functionality of an MP3 player. Other input/output and control devices can also be used.

Memory interface 602 can be coupled to memory 650. Memory 650 can include high-speed random access memory and/or non-volatile memory, such as one or more magnetic disk storage devices, one or more optical storage devices and/or flash memory (e.g., NAND, NOR). Memory 650 can store operating system 652, such as the iOS operating system developed by Apple Inc. of Cupertino, California. Operating system 652 may include instructions for handling basic system services and for performing hardware dependent tasks. In some implementations, operating system 652 can include a kernel (e.g., UNIX kernel).

Memory 650 may also store communication instructions 654 to facilitate communicating with one or more additional devices, one or more computers and/or one or more servers, such as, for example, instructions for implementing a software stack for wired or wireless communications with other devices. Memory 650 may include graphical user interface instructions 656 to facilitate graphic user interface processing; sensor processing instructions 658 to facilitate sensor-related processing and functions; phone instructions 660 to facilitate phone-related processes and functions; electronic messaging instructions 662 to facilitate electronic-messaging related processes and functions; web browsing instructions 664 to facilitate web browsing-related processes and functions; media processing instructions 666 to facilitate media processing-related processes and functions; GNSS/Location instructions 668 to facilitate generic GNSS and location-related processes and instructions; and gesture recognition instructions 670 that implement the gesture recognition processes described in reference to FIGS. 2-5. Memory 650 further includes other application instructions 672 including but not limited to instructions for applications that respond to finger/hand gestures.

Each of the above identified instructions and applications can correspond to a set of instructions for performing one or more functions described above. These instructions need not be implemented as separate software programs, procedures, or modules. Memory 650 can include additional instructions or fewer instructions. Furthermore, various functions of the mobile device may be implemented in hardware and/or in software, including in one or more signal processing and/or application specific integrated circuits.

### Example Architecture for Customized Gesture Prediction Head

FIG. 7A to 7C is a block diagram of a training data processing pipeline 700 of training a gesture customized prediction head (e.g., gesture prediction head 216), according to an embodiment. Because training a model with less than, e.g., 10 samples is challenging, the model can easily fall into an overfitting problem. It is also hard to prevent false-positives as the model does not have enough "positive" samples (e.g., gesture samples) to learn from. To address this issue, training data processing pipeline 700 is employed to increase the amount of training data above what is provided by users. Pipeline 700 starts with a short data sequence (e.g., 3 shots) recorded by a user and steps through data segmentation 701, data augmentation 702 and data synthesis 703 prior to training. In an embodiment, the training process is enhanced by adversarial regularization 704. Each step is described in turn below.

### Data Segmentation

Before actual data processing, it is noteworthy that there is no readily available sample. When users record data of their new customized gestures, they can either do gestures consecutively in a row, or follow some instructions to do one gesture at a time and repeat several times, depending on the interaction design. In either way, it may be undesirable to ask users to provide the exact start and end timestamp of the gesture. Therefore, the signal sequence is segmented to obtain data samples.

In an embodiment, the sensor signals (e.g., accelerometer, gyro, PPG signals) are input into respective middle bandpass filters. A peak detection algorithm is applied to the output signals of middle bandpass filters (e.g., 8-32 Hz) to identify potential moments of performing hand gestures. In an embodiment, the sum of the magnitude of the filtered accelerometer and gyroscope signals is calculated, and an absolute moving average window (e.g., 1 sec) is applied to smooth the data. In an embodiment, a peak detection method is used to find local maxima by comparing neighboring values (e.g., with distance threshold as 1 sec), where a peak is ignored if it is lower than an overall average of signal magnitude. If any time reference is available (e.g., a countdown mechanism), peaks can be further filtered according to the reference. An n-sec window (e.g., 1 sec) is centered at these potential peaks, and input into a feature extraction part of a pre-trained model. In an embodiment, a distance matrix (e.g., a Euclidean distance matrix) of normalized embedding vectors is computed and the peaks whose embeddings are far from other embeddings based on a empirically set threshold (e.g., 0.8) are removed. In this manner, pronounced, repetitive hand movement periods that correspond to the target gestures are segmented from the sensor signals.

Once the peaks are determined, a n-sec window (e.g., 1.5 sec) is centered at each final peak to ensure that a gesture is fully covered by the window. Data augmentation techniques are then applied to these windows.

### Data Augmentation

After data segmentation, several data augmentation techniques are used to generate a larger number of samples. In an embodiment, three time series data augmentation techniques and all their combinations (e.g., seven combinations (23 - 1) in total) are used to generate positive samples: 1) zooming, to simulate different gesture speed, randomly chosen from ×0.9 to ×1; 2) scaling, to simulate different gesture strength, with the scaling factor s ~ N (1, 0.22), s ∈ [0, 2]; and 3) time-warping, to simulate gesture temporal variance, with 2 interpolation knots and warping randomness w~N(1,0.052),*w*∈[0,2].

In an embodiment, three augmentation techniques are employed to generate negative data: 1) cutting out by masking a random portion (e.g., 0.5 sec) of signals by zero; 2) reversing signals; and 3) shuffling by slicing signals into pieces (e.g., 0.1 sec pieces) and generating a random permutation. These augmentations are typically used in other machine learning tasks to augment positive data; however, in this embodiment the techniques are used to augment negative data to ensure the model only recognizes valid gestures. The positive augmentation techniques describe above can also be applied to the negative data to generate more negative samples.

### Data Synthesis

Although the data augmentation can generate signals with larger variance from the data recorded by users, these augmented data may not be close to the actual gesture variance introduced by natural human behavior. Therefore, more data can be synthesized from both the raw signals and the augmented signals that simulate the natural motion variance. In an embodiment, a Δ-encoder is trained, which is a self-supervised encoder-decoder model that can capture the difference between two samples (i.e., Δ) belonging to the same gesture, and use the difference to synthesize more new gesture samples.

**In** an embodiment, a Δ-encoder is trained as follows. The Δ-encoder takes two samples (sampleInput and sampleRef) from the same class as the input, feeds sampleInput through a few neural network layers to be a very small embedding called Δ-vector (similar to a typical autoencoder, and then use the Δ-vector and the sampleRef to reconstruct sampleInput. The intuition comes from that the size of Δ-vector is so small that it focuses on capturing the difference between sampleInput and sampleRef, which is then used to rebuild sampleInput with sampleRef as the reference. After the Δ-encoder is trained, it can take another sample from the new class as a new sampleRef, and generate a new sample of the same class with a Δ-vector. This Δ-vector can either be obtained by feeding any existing sample from other classes through the encoder, or randomly generated.

**In** an embodiment, data of the existing four gestures previously described above (pinching the thumb and index finger together, clenching the hand into a fist, tapping one or more fingers on a surface and knocking a fist on a surface) are used to train a Δ-encoder. During the training, two samples are randomly drawn from the same gesture and the same user to ensure that the model captures the within-user variance instead of the between-user variance. the feature embeddings (e.g., of length 120) are used as the input and the output of the Δ-encoder to save computation cost. In an embodiment, both the encoder and decoder have one hidden layer with a size of 4096 and uses leaky ReLU (*α* = 0.3) as the activation function. In an embodiment, the size of Δ-vector is set as 5.

Using the training set from the four gestures described above, the model is trained with, e.g., 200 epochs and has a, e.g., 0.5 exponential decay on the learning rate every 30 epochs. The epoch with the best results on the validation set is saved. The Δ-vectors from the testing set of the four gestures are also calculated and saved to be used to generate new samples. In real-time, when the customized gesture data goes through the augmentation stage, the Δ-encoder is used to generate extra samples of the customized gestures (both positive and negative data) that contain more natural gesture variance.

### Adversarial Training Regularization

After the data augmentation and data synthesis, a large amount of data with appropriate variance is obtained to train the gesture prediction head. To further improve the robustness of the model, in some embodiments the adversarial training regularization is employed when learning the model. Adversarial regularization is used to train a model with adversarial-perturbed data (perturbed towards the decision boundary or inverse gradient decent so that the training process becomes harder) in addition to the original training data. It can prevent the model from overfitting and classify the data points close to the boundary more robustly. In an embodiment, customized gesture data from the same user tend to be blended with the existing four gestures near the boundary. Adversarial regularization can help to enhance classification performance, especially for the purpose of reducing false-positive. In an embodiment, the adversarial regularization loss weight and the reverse gradient step size are set to 0.2.

Accordingly, through a series of data segmentation, data augmentation, data synthesis, and adversarial training, a robust prediction head can be learned for each new user that can accurately recognize their customized gestures with a low false-positive rate.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

As described above, some aspects of the subject matter of this specification include gathering and use of data available from various sources to improve services a mobile device can provide to a user. The present disclosure contemplates that in some instances, this gathered data may identify a particular location or an address based on device usage. Such personal information data can include location-based data, addresses, subscriber account identifiers, or other identifying information.

The present disclosure further contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure. For example, personal information from users should be collected for legitimate and reasonable uses of the entity and not shared or sold outside of those legitimate uses. Further, such collection should occur only after receiving the informed consent of the users. Additionally, such entities would take any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices.

**In** the case of advertisement delivery services, the present disclosure also contemplates embodiments in which users selectively block the use of, or access to, personal information data. That is, the present disclosure contemplates that hardware and/or software elements can be provided to prevent or block access to such personal information data. For example, in the case of advertisement delivery services, the present technology can be configured to allow users to select to "opt in" or "opt out" of participation in the collection of personal information data during registration for services.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, content can be selected and delivered to users by inferring preferences based on non-personal information data or a bare minimum amount of personal information, such as the content being requested by the device associated with a user, other non-personal information available to the content delivery services, or publicly available information.

## Claims

1. A method comprising:
receiving, with at least one processor (604), sensor data indicative of a customized gesture made by a user, the sensor data obtained from at least one sensor of a wearable device (101) worn on a limb of the user;
generating, with at least one processor (604), a current encoding (404) of features extracted from the sensor data using a machine learning model with the features as input, where the machine learning model includes a pre-trained gesture recognition model for predicting a gesture class based on a set of known gestures and an additional prediction head for predicting customized gestures;
generating, with at least one processor (604), similarity metrics (405) between the current encoding (404) and each encoding in a set of previously generated encodings (403) by the machine learning model for gestures;
generating, with at least one processor (604), similarity scores (407) based on the similarity metrics (405);
predicting, with the at least one processor (604), the customized gestures made by the user using the machine learning model with the similarity scores (407) as input; and
performing, with at least one processor (604), an action on the wearable device (101) or other device based on the predicted customized gestures.

2. The method of claim 1, wherein the limb is a wrist of the user, and the sensor data is obtained from a combination of a bio signal and at least one motion signal.

3. The method of claim 2, wherein the bio signal is a photoplethysmography, PPG, signal and the at least one motion signal is acceleration or angular rate.

4. The method of claim 1, wherein the similarity metrics (405) are distance metrics.

5. The method of claim 1, wherein the machine learning model is a neural network.

6. The method of claim 1, wherein the similarity scores (407) are generated by a neural network.

7. The method of claim 6, wherein the neural network is a deep neural network that includes a sigmoid activation function.

8. The method of claim 1, wherein the action corresponds to navigating a user interface on the wearable device (101) or other device.

9. The method of claim 1, wherein the machine learning model is a neural network trained using sample data for pairs of gestures obtained from a known set of gestures, where each gesture in the pair is annotated with a label indicating that the gesture is from a same class or a different class, and a feature vector for each gesture in the pair is separately encoded using the machine learning model.

10. The method of claim 9, wherein the machine learning model uses a different loss function for each gesture in each pair during training.

11. A system comprising:
at least one processor (604);
memory (650) storing instructions, that when executed by the at least one processor (604), cause the at least one processor (604) to perform operations comprising:
receiving sensor data indicative of a customized gesture made by a user, the sensor data obtained from at least one sensor of a wearable device (101) worn on a limb of the user;
generating a current encoding (404) of features extracted from the sensor data using a machine learning model with the features as input, where the machine learning model includes a pre-trained gesture recognition model for predicting a gesture class based on a set of known gestures and an additional prediction head for predicting customized gestures;
generating similarity metrics (405) between the current encoding (404) and each encoding in a set of previously generated encodings (403) by the machine learning model for known gestures;
generating similarity scores (407) based on the similarity metrics (405);
predicting the customized gestures made by the user using the machine learning model with the similarity scores (407) as input; and
performing, with at least one processor (604), an action on the wearable device (101) or other device based on the predicted customized gestures.

12. The system of claim 11, wherein the limb is a wrist of the user, and the sensor data is obtained from a combination of a bio signal and at least one motion signal.

13. The system of claim 12, wherein the bio signal is a photoplethysmography, PPG, signal and the motion signal is at least one of acceleration or angular rate.

14. The system of claim 11, wherein the similarity metrics (405) are distance metrics.

15. The system of claim 11, wherein the machine learning model is a neural network.

16. The system of claim 11, wherein the similarity scores (407) are generated by a neural network.

17. The system of claim 16, wherein the neural network is a deep neural network that includes a sigmoid activation function.

18. The system of claim 17, wherein the predetermined action corresponds to navigating a user interface on the wearable device (101) or other device.

19. The system of claim 11, wherein the machine learning model is a neural network trained using sample data for pairs of gestures obtained from a known set of gestures, where each gesture in the pair is annotated with a label indicating that the gesture is from a same class or a different class, and a feature vector for each gesture in the pair is separately encoded using the machine learning model.

20. The system of claim 19, wherein the machine learning model uses a different loss function for each gesture in each pair during training.

## Patentansprüche

1. Verfahren, aufweisend:
Empfangen, mit mindestens einem Prozessor (604), von Sensordaten, die eine von einem Benutzer vorgenommene angepasste Geste anzeigen, wobei die Sensordaten von mindestens einem Sensor einer tragbaren Vorrichtung (101) erhalten werden, die an einer Gliedmaße des Benutzers getragen wird;
Erzeugen, mit mindestens einem Prozessor (604), einer aktuellen Codierung (404) von aus den Sensordaten extrahierten Merkmalen unter Verwendung eines maschinellen Lernmodells mit den Merkmalen als Eingabe, wobei das maschinelle Lernmodell ein vortrainiertes Gestenerkennungsmodell zum Vorhersagen einer Gestenklasse basierend auf einem Satz bekannter Gesten und einen zusätzlichen Vorhersagekopf zum Vorhersagen angepasster Gesten beinhaltet;
Erzeugen, mit mindestens einem Prozessor (604), von Ähnlichkeitsmetriken (405) zwischen der aktuellen Codierung (404) und jeder Codierung in einem Satz zuvor erzeugter Codierungen (403) durch das maschinelle Lernmodell für Gesten;
Erzeugen, mit mindestens einem Prozessor (604), von Ähnlichkeitsbewertungen (407) basierend auf den Ähnlichkeitsmetriken (405);
Vorhersagen, mit dem mindestens einen Prozessor (604), der von dem Benutzer vorgenommenen angepassten Gesten unter Verwendung des maschinellen Lernmodells mit den Ähnlichkeitsbewertungen (407) als Eingabe; und
Durchführen, mit mindestens einem Prozessor (604), einer Aktion an der tragbaren Vorrichtung (101) oder einer anderen Vorrichtung basierend auf den vorhergesagten angepassten Gesten.

2. Verfahren nach Anspruch 1, wobei die Gliedmaße ein Handgelenk des Benutzers ist und die Sensordaten aus einer Kombination eines Biosignals und mindestens eines Bewegungssignals erhalten werden.

3. Verfahren nach Anspruch 2, wobei das Biosignal ein Photoplethysmographie-, PPG-, Signal ist und das mindestens eine Bewegungssignal Beschleunigung oder Winkelrate ist.

4. Verfahren nach Anspruch 1, wobei die Ähnlichkeitsmetriken (405) Abstandsmetriken sind.

5. Verfahren nach Anspruch 1, wobei das Maschinenlernmodell ein neuronales Netzwerk ist.

6. Verfahren nach Anspruch 1, wobei die Ähnlichkeitsbewertungen (407) durch ein neuronales Netzwerk erzeugt werden.

7. Verfahren nach Anspruch 6, wobei das neuronale Netzwerk ein tiefes neuronales Netzwerk ist, das eine Sigmoid-Aktivierungsfunktion enthält.

8. Verfahren nach Anspruch 1, wobei die Aktion dem Navigieren einer Benutzerschnittstelle an der tragbaren Vorrichtung (101) oder einer anderen Vorrichtung entspricht.

9. Verfahren nach Anspruch 1, wobei das maschinelle Lernmodell ein neuronales Netzwerk ist, das unter Verwendung von Musterdaten für Paare von Gesten trainiert wird, die aus einem bekannten Satz von Gesten erhalten werden, wobei jede Geste in dem Paar mit einer Beschriftung versehen ist, die angibt, dass die Geste aus einer gleichen Klasse oder einer anderen Klasse stammt, und ein Merkmalsvektor für jede Geste in dem Paar unter Verwendung des maschinellen Lernmodells separat codiert wird.

10. Verfahren nach Anspruch 9, wobei das maschinelle Lernmodell eine andere Verlustfunktion für jede Geste in jedem Paar während des Trainings verwendet.

11. System, aufweisend:
mindestens einen Prozessor (604);
einen Speicher (650), der Anweisungen speichert, die, wenn sie von dem mindestens einen Prozessor (604) ausgeführt werden, den mindestens einen Prozessor (604) veranlassen, Operationen durchzuführen, aufweisend:
Empfangen von Sensordaten, die eine von einem Benutzer vorgenommene angepasste Geste anzeigen, wobei die Sensordaten von mindestens einem Sensor einer tragbaren Vorrichtung (101) erhalten werden, die an einer Gliedmaße des Benutzers getragen wird;
Erzeugen einer aktuellen Codierung (404) von aus den Sensordaten extrahierten Merkmalen unter Verwendung eines maschinellen Lernmodells mit den Merkmalen als Eingabe, wobei das maschinelle Lernmodell ein vortrainiertes Gestenerkennungsmodell zum Vorhersagen einer Gestenklasse basierend auf einem Satz bekannter Gesten und einen zusätzlichen Vorhersagekopf zum Vorhersagen angepasster Gesten beinhaltet;
Erzeugen von Ähnlichkeitsmetriken (405) zwischen der aktuellen Codierung (404) und jeder Codierung in einem Satz zuvor erzeugter Codierungen (403) durch das maschinelle Lernmodell für bekannte Gesten;
Erzeugen von Ähnlichkeitsbewertungen (407) basierend auf den Ähnlichkeitsmetriken (405);
Vorhersagen der von dem Benutzer vorgenommenen angepassten Gesten unter Verwendung des maschinellen Lernmodells mit den Ähnlichkeitsbewertungen (407) als Eingabe; und
Durchführen, mit mindestens einem Prozessor (604), einer Aktion an der tragbaren Vorrichtung (101) oder einer anderen Vorrichtung basierend auf den vorhergesagten angepassten Gesten.

12. System nach Anspruch 11, wobei die Gliedmaße ein Handgelenk des Benutzers ist und die Sensordaten aus einer Kombination eines Biosignals und mindestens eines Bewegungssignals erhalten werden.

13. System nach Anspruch 12, wobei das Biosignal ein Photoplethysmographie-, PPG-, Signal ist und das Bewegungssignal mindestens eines von Beschleunigung oder Winkelrate ist.

14. System nach Anspruch 11, wobei die Ähnlichkeitsmetriken (405) Abstandsmetriken sind.

15. System nach Anspruch 11, wobei das Maschinenlernmodell ein neuronales Netzwerk ist.

16. System nach Anspruch 11, wobei die Ähnlichkeitsbewertungen (407) durch ein neuronales Netzwerk erzeugt werden.

17. System nach Anspruch 16, wobei das neuronale Netzwerk ein tiefes neuronales Netzwerk ist, das eine Sigmoid-Aktivierungsfunktion enthält.

18. System nach Anspruch 17, wobei die vorbestimmte Aktion dem Navigieren einer Benutzerschnittstelle an der tragbaren Vorrichtung (101) oder einer anderen Vorrichtung entspricht.

19. System nach Anspruch 11, wobei das maschinelle Lernmodell ein neuronales Netzwerk ist, das unter Verwendung von Musterdaten für Paare von Gesten trainiert wird, die aus einem bekannten Satz von Gesten erhalten werden, wobei jede Geste in dem Paar mit einer Beschriftung versehen ist, die angibt, dass die Geste aus einer Geste in dem Paar unter Verwendung des maschinellen Lernmodells separat codiert wird.

20. System nach Anspruch 19, wobei das maschinelle Lernmodell eine andere Verlustfunktion für jede Geste in jedem Paar während des Trainings verwendet.

## Revendications

1. Un procédé comprenant :
la réception, avec au moins un processeur (604), de données de capteur représentatives d'un geste personnalisé effectué par un utilisateur, les données de capteur étant obtenues depuis au moins un capteur d'un dispositif pouvant être porté sur soi (101) porté sur un membre de l'utilisateur ;
la génération, avec au moins un processeur (604), d'un codage courant (404) de caractéristiques extraites des données de capteur en utilisant un modèle d'apprentissage machine avec les caractéristiques en entrée, le modèle d'apprentissage machine comprenant un modèle de reconnaissance de geste pré-entraîné pour prédire une classe de gestes sur la base d'un ensemble de gestes connus et d'une tête de prédiction additionnelle pour prédire des gestes personnalisés ;
la génération, avec au moins un processeur (604), de métriques de similarité (405) entre le codage courant (404) et chaque codage d'un ensemble de codages précédemment générés (403) par le modèle d'apprentissage machine pour des gestes ;
la génération, avec au moins un processeur (604), de scores de similarité (407) basés sur les métriques de similarité (405) ;
la prédiction, avec l'au moins un processeur (604), des gestes personnalisés effectués par l'utilisateur en utilisant le modèle d'apprentissage machine avec en entrée les scores de similarité (407) ; et
l'exécution, avec au moins un processeur (604), d'une action sur le dispositif pouvant être porté sur soi (101) ou tout autre dispositif, sur la base des gestes personnalisés prédits.

2. Le procédé de la revendication 1, dans lequel le membre est un poignet de l'utilisateur, et les données de capteur sont obtenues à partir d'une combinaison d'un signal biométrique et d'au moins un signal de mouvement.

3. Le procédé de la revendication 2, dans lequel le signal biométrique est un signal photopléthysmographique, PPG, et l'au moins un signal de mouvement est une accélération ou une vitesse angulaire.

4. Le procédé de la revendication 1, dans lequel les métriques de similarité (405) sont des métriques de distance.

5. Le procédé de la revendication 1, dans lequel le modèle d'apprentissage machine est un réseau de neurones.

6. Le procédé de la revendication 1, dans lequel les scores de similarité (407) sont générés par un réseau de neurones.

7. Le procédé de la revendication 6, dans lequel le réseau de neurones est un réseau de neurones profond qui comprend une fonction d'activation sigmoïdale.

8. Le procédé de la revendication 1, dans lequel l'action correspond à une navigation d'une interface utilisateur sur le dispositif pouvant être porté sur soi (101) ou un autre dispositif.

9. Le procédé de la revendication 1, dans lequel le modèle d'apprentissage machine est un réseau de neurones entraînés en utilisant des échantillons de données pour des paires de gestes obtenues à partir d'un ensemble connu de gestes, chaque geste de la paire étant annoté avec un marqueur indiquant que le geste est d'une même classe ou d'une classe différente, et un vecteur de caractéristiques pour chaque geste de la paire est codé séparément en utilisant le modèle d'apprentissage machine.

10. Le procédé de la revendication 9, dans lequel le modèle d'apprentissage machine utilise pendant l'entraînement une fonction de pertes différente pour chaque geste de chaque paire.

11. Un système comprenant :
au moins un processeur (604) ;
une mémoire (650) stockant des instructions qui, lorsqu'elles sont exécutées par l'au moins un processeur (604), font en sorte que l'au moins un processeur (604) effectue des opérations comprenant :
la réception de données de capteur représentatives d'un geste personnalisé effectué par un utilisateur, les données de capteur étant obtenues depuis au moins un capteur d'un dispositif pouvant être porté sur soi (101) porté sur un membre de l'utilisateur ;
la génération d'un codage courant (404) de caractéristiques extraites des données de capteur en utilisant un modèle d'apprentissage machine avec les caractéristiques en entrée, le modèle d'apprentissage machine comprenant un modèle de reconnaissance de geste pré-entraîné pour prédire une classe de gestes sur la base d'un ensemble de gestes connus et d'une tête de prédiction additionnelle pour prédire des gestes personnalisés ;
la génération de métriques de similarité (405) entre le codage courant (404) et chaque codage d'un ensemble de codages précédemment générés (403) par le modèle d'apprentissage machine pour des gestes ;
la génération de scores de similarité (407) basés sur les métriques de similarité (405) ;
la prédiction des gestes personnalisés effectués par l'utilisateur en utilisant le modèle d'apprentissage machine avec en entrée les scores de similarité (407) ; et
l'exécution, avec au moins un processeur (604), d'une action sur le dispositif pouvant être porté sur soi (101) ou tout autre dispositif, sur la base des gestes personnalisés prédits.

12. Le système de la revendication 11, dans lequel le membre est un poignet de l'utilisateur, et les données de capteur sont obtenues à partir d'une combinaison d'un signal biométrique et d'au moins un signal de mouvement.

13. Le système de la revendication 12, dans lequel le signal biométrique est un signal photopléthysmographique, PPG, et l'au moins un signal de mouvement est une accélération ou une vitesse angulaire.

14. Le système de la revendication 11, dans lequel les métriques de similarité (405) sont des métriques de distance.

15. Le système de la revendication 11, dans lequel le modèle d'apprentissage machine est un réseau de neurones.

16. Le système de la revendication 11, dans lequel les scores de similarité (407) sont générés par un réseau de neurones.

17. Le système de la revendication 16, dans lequel le réseau de neurones est un réseau de neurones profond qui comprend une fonction d'activation sigmoïdale.

18. Le système de la revendication 11, dans lequel l'action prédéterminée correspond à une navigation d'une interface utilisateur sur le dispositif pouvant être porté sur soi (101) ou un autre dispositif.

19. Le système de la revendication 11, dans lequel le modèle d'apprentissage machine est un réseau de neurones entraînés en utilisant des échantillons de données pour des paires de gestes obtenues à partir d'un ensemble connu de gestes, chaque geste de la paire étant annoté avec un marqueur indiquant que le geste est d'une même classe ou d'une classe différente, et un vecteur de caractéristiques pour chaque geste de la paire est codé séparément en utilisant le modèle d'apprentissage machine.

20. Le système de la revendication 19, dans lequel le modèle d'apprentissage machine utilise pendant l'entraînement une fonction de pertes différente pour chaque geste de chaque paire.
